# EUROPEAN PATENT APPLICATION

(11) **EP 1 920 663 A1**
(43) Date of publication of application: **14.05.2008**
(21) Application number: 07021787.2
(22) Date of filing: 09.11.2007
(51) Int. Cl.: A23L 1/00, A61K 9/50

(54) **Coating for oxygen sensitive materials**

(30) Priority: 10.11.2006 US 558510
(71) Applicant: National Starch and Chemical Investment Holding Corporation, New Castle, Delaware 19720 (US)
(72) Inventor: Ponginebbi, Lucia, Monmouth Junction New Jersey 08852 (US); Puglisi, Christine, Mountainside New Jersey 07092 (US)
(74) Representative: Held, Stephan

(57) **Abstract**

The invention relates to coating and encapsulation composition useful for protecting core material from oxidative and/or hydrolytic damage. The coating composition comprises a lipid polymer and a lipid-based anti-oxidant, wherein the anti-oxidant is soluble in the lipid polymer.

## Description

### FIELD OF THE INVENTION

The invention relates to a protective coating for core material by the addition of a lipid-based anti-oxidant in a lipid-based polymer system. In particular, the coating is useful in protecting core material that otherwise would degrade in the presence of oxygen and/or water.

### BACKGROUND OF THE INVENTION

Coating compositions are used to coat or encapsulate a core material in order to provide a protective film or provide delay release properties. In many industries, ingredients exhibit significant decrease or degradation in their desired properties due to oxidation and/or hydrolysis. For example, the food, pharmaceutical, cosmeceutical and nutraceutical industries utilize a significant number of substances that are prone to oxidation. As the substances oxidize, the desired properties of the material may be damaged, i.e., may decrease, degrade or eliminate, or the properties of the material can be changed entirely to provide undesired properties. In these instances, oxidation protection is required in order to ensure that the substances provide their desired effects at the time of their ultimate use.

There is an ongoing demand for effective means to counter oxidation and/or hydrolytic processes. The need thus exists in the art to provide effective protection for a wide range of core materials, to avoid oxidation damage and hydrolytic degradation in order to preserve the desired positive properties of the core material. The current invention addresses this need in the art.

### SUMMARY OF THE INVENTION

The invention relates to a coating composition useful for protecting an active material, hereinafter a core material, from oxidative and/or hydrolytic damage. In particular, the present invention provides coating materials and methods, which protect the encapsulated core material from e.g. damage by inhibiting oxygen diffusion through the encapsulating material, and thus protect the encapsulated core materials from damaging oxidation.

One embodiment provides coating composition that decrease oxygen permeability, or even oxygen impermeability, to the core substance. The coating composition comprises a lipid polymer and a lipid-based anti-oxidant. The lipid-based anti-oxidant combined with the lipid polymer enhances the oxygen barrier properties of the coating. The lipid polymer preferred for use can be selected from a class of waxes and lipids. A particularly preferred lipid-based anti-oxidant is ascorbyl palmitate.

In one embodiment the coating comprises a lipid polymer and a lipid-based anti-oxidant that provides a barrier to protect the core substance, wherein the barrier decreases oxygen permeability through the coating. The barrier for the core substance comprises a lipid polymer and a lipid-based anti-oxidant.

In another embodiment, the present invention provides an encapsulated material, comprising: a core component, and a shell component encapsulating the core component. The shell component comprises a lipid polymer material and a lipid-based anti-oxidant which decreases oxygen permeability through the shell component.

Yet another aspect of the invention is directed to a method of forming the coated materials, comprising suspending the core material on a jet of air, spraying the coating component onto the core material, wherein the coating material is made of lipid polymer and lipid-based anti-oxidant, and solidifying the coating component to form a coated material.

### BRIEF DESCRIPTION OF THE FIGURES

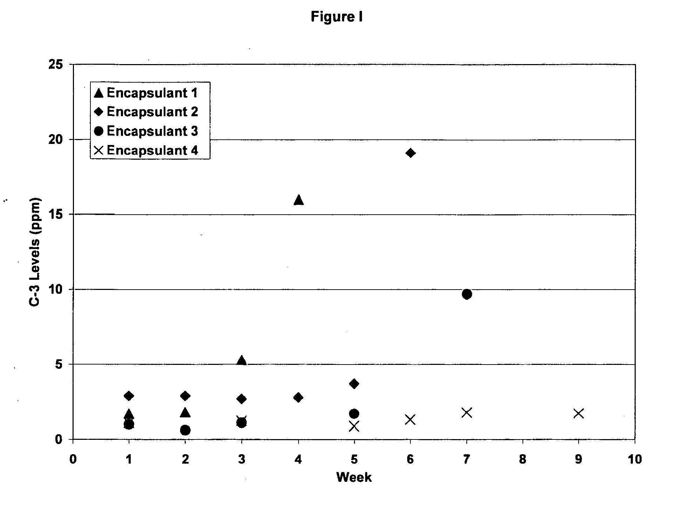

Figure 1 shows the amount of free C-3 aldehydes released from Encapsulants 1(▲), 2 (◆), 3 (•) and 4(x).

### DETAILED DESCRIPTION OF THE INVENTION

Coatings are used to encapsulate a desired core material, particularly oxygen and/or water sensitive materials. According to the present invention, a coating material is provided that comprises a lipid polymer material and a lipid-based anti-oxidant.

Core material, ingredient, active and substance are used interchangeably throughout this disclosure and refer to component which is encapsulated by another component. A core material, ingredient, active or substance is a component having a desirable or perceived property, such as food, pharmaceutical, physiological or fragrance.

As used herein, the term "lipid-based polymer" refers to hydrocarbon based compounds that are soluble in non-polar solvents and relatively insoluble in non-polar compounds.

The term "lipid based anti-oxidant" as used herein refers to a chemical that is soluble in non-polar solvents, relatively insoluble in water and which will act to reduce the rate of oxidation in a given system.

The term "damage" as used herein means oxidative decomposition of the active component(s), where the active may decrease, degrade, or eliminate, or the properties of the material can be changed entirely to provide undesired properties.

The invention provides products and methods for protecting oxygen and/or water sensitive materials, as well as other materials, from the destructive contact with oxygen and/or water or other permeating species. For example, in the case of oils and fats, it has been conventionally known to try to encapsulate the oils and fats to protect them from oxidation and hydrolysis. Such oils and fats have conventionally been protected by either using physical means to keep the oils and fats away from oxygen or water or storing them at reduced temperature.

The present invention provides a means to protect core materials from oxygen and/or water and other detrimental species. The addition of a lipid-based anti-oxidant to lipid polymer changes the performance of the coating layer. The result is a significant decrease in permeability of oxygen through the coating shell.

The food, pharmaceutical, cosmeceutical and nutraceutical industries utilize a significant number of substances that are prone to oxidation. As the substances oxidize, the desired properties of the core material can be damaged, decreased or degraded, or even eliminated, or the properties of the substance can be changed entirely to provide undesired properties. Non-limiting examples of core materials include fats, oils, volatile molecules, flavours, vitamins, fragrances, enzymes or minerals. Oils, such as omega-3 fatty acids, are highly unstable and subject to oxidation when exposed to air. This oxidation process causes the oil to turn rancid, giving an unpleasant taste and smell, and/or undesirable health benefits. The encapsulation process protects the stability of core materials following processing and during long term storage.

While lipid polymers are known to exhibit limited oxygen impermeability, the use of lipid polymers alone is often not sufficient to provide desired oxygen and/or water barrier properties for many applications. For example, some lipid polymers exhibit undesirably high oxygen and/or water transmission rates, and thus are unsuitable for oxygen and/or water sensitive materials. Alternatively, depending upon the use of the resultant material, various polymers cannot be selected. For example, in the food, pharmaceutical, cosmeceutical and nutraceutical areas, as well as in the area of consumer goods, a polymer must be selected that is appropriate for ingestion or for contact with tissues. In these and other areas, proper selection of the encapsulating polymer must thus account for not only the material to be encapsulated and the oxygen and/or water permeability through the polymer, but also the polymer's suitability for an end-use application.

According to the present invention, the coating material comprises a lipid polymer and a lipid-based anti-oxidant. Suitable lipid polymer materials used as a coating material in the present invention can be selected from any suitable known or after-developed polymers. In certain embodiments, the lipid polymer will be selected from, but is not limited to, wax, such as carnauba wax, and paraffin wax, and the like; partially hydrogenated oil, such as partially hydrogenated palm oil, partially hydrogenated cottonseed oil, and the like; fats; proteins, such as whey, soy, corn, and the like; gums; alginate; carrageenan; cellulose derivatives, such as cellulose, ethyl cellulose, cellulose acetate trimellitate, cellulose acetate phthalate, methyl cellulose, and the like; rosins; synthetic polymer materials, such as nylons, polyurethanes, polyglycolic acid, polyethylene glycol, polyamides, acetates, triacetates, polyacrylics, polyacrylonitile, including polyethylenes and polypropylenes, polyesters, polyvinyl alcohol, polyvinyl acetate, and the like; fluorinated polymers such as polytetrafluoroethylene; and mixtures thereof. Of these, several of the materials such as waxes, partially hydrogenated oils, fats, proteins, cellulose derivatives, gums, alginate, and carrageenan are preferred for applications in the food, pharmaceutical and consumer goods areas, while other materials, such as the synthetic polymer materials are preferred for other applications.

The addition of lipid-based anti-oxidant into lipid polymer protects the core material against degradation and ensures long-term stability. The coating made from the lipid-based anti-oxidant and lipid polymer increases the shelf-life and usefulness of the encapsulated core material, such as any of the various oxygen and/or water sensitive compounds.

Representative antioxidants that may be used in the practice of the invention include: butylated hydroxyanisole (BHA), butylated hydroxytoluene (BHT), tert-butylhydroquinone (TBHQ), ascorbyl palmitate, tocopherols, ethoxyquinone, propyl gallate, rosemary extract, grapeseed extract, ascorbyl stearate and hydroquinone. Combinations of antioxidants may be used to protect the core materials due to the different mechanisms of degradation to which various polymers are subject.

Preferably, the lipid-based anti-oxidant is added from about 0.005% to about 5%, more preferably from about 0.02% to about 0.5% by weight of the polymer.

According to the present invention, the lipid-based anti-oxidant can be incorporated into the lipid polymer matrix. Lipid polymers provide a barrier to moisture however their oxygen barrier properties are poor. The addition of the lipid-based anti-oxidant into the lipid polymer coating effectively minimizes oxygen diffusion into the core material. The addition of lipid-based anti-oxidant changes the characteristic of the lipid polymer into a good moisture and oxygen barrier. Hence, the addition of a lipid-based anti-oxidant into the lipid polymer coating changes the ability of the coating to function as both a moisture and an oxygen barrier.

Suitable methods for forming the encapsulated core materials include physio-mechanical techniques. Non-limiting methods to encapsulate core materials include spray-drying, multiple nozzle spraying, centrifugal technique, vacuum encapsulation, electrostatic encapsulation and fluid-bed coating. A preferred method for forming the encapsulated particle is a fluidized-bed coating method. In the fluidized-bed process, the core materials are suspended on a jet of air whereupon the coating is sprayed onto the core material. The process of suspending, spraying, and cooling is repeated until the coat develops a desired thickness. The coating is sprayed onto the core material until about 20 to about 70 percent by coating weight, based on core weight, is used to encapsulate the core material. The resultant shell generally has a thickness of from about 1 µm to about 1,000 µm, preferably from about 5 µm to about 500 µm, and more preferably from about 10 µm to about 200 µm.

The following embodiments are presented to further illustrate and explain the present invention and should not be taken as limiting in any regard.
1. A coating composition comprising: a lipid polymer; and a lipid-based anti-oxidant, wherein the anti-oxidant is soluble in the lipid polymer.
2. The coating composition of embodiment 1 wherein the polymer is selected from the group consisting of waxes, partially hydrogenated oil, fats, gums, celluloses derivatives, proteins, synthetic polymer materials, alginate, carrageenan and mixtures thereof.
3. The coating composition of embodiment 1 wherein the anti-oxidant is selected from the group consisting of butylated hydroxyanisole (BHA), butylated hydroxytoluene (BHT), tert-butylhydroquinone (TBHQ), ascorbyl palmitate, tocopherols, ethoxyquinone, propyl gallate, rosemary extract, grapeseed extract, ascorbyl stearate, hydroquinone and mixtures thereof.
4. The coating composition of embodiment 3, wherein said antioxidant is present in an amount of about 0.005 to about 5 percent by weight of the lipid polymer.
5. The coating composition of embodiment 4, wherein said antioxidant is present in an amount of about 0.02 to about 0.5 percent by weight of the lipid polymer.
6. An encapsulated material, comprising: a core component; and a shell component encapsulating said core component, wherein said shell component comprises a lipid polymer material and a lipid-based anti-oxidant.
7. The encapsulated material of embodiment 6, wherein said core component is at least one of oxygen sensitive or water sensitive.
8. The encapsulated material of embodiment 7, wherein said core component is selected from the group consisting of unsaturated fatty acids, betacarotene, lutein, zeazanthin, iron salts, copper salts, selenium salts, flavonoids, coenzyme Q10, herbs, spices, flavorants, extracts, protein and peptide drugs, amino acids and amino acid residues, surfactants, enzymes, peroxides, fragrances, catalysts, vitamins, nutritional supplements, minerals, herbal products, food additives and mixtures thereof.
9. The encapsulated material of embodiment 6 wherein said lipid polymer material is selected from the group consisting of waxes, partially hydrogenated oil, fats, gums, celluloses derivatives, proteins, synthetic polymer materials, alginate, carrageenan and mixtures thereof.
10. The encapsulated material of embodiment 6, wherein said anti-oxidant is selected from the group consisting of butylated hydroxyanisole (BHA), butylated hydroxytoluene (BHT), tert-butylhydroquinone (TBHQ), ascorbyl palmitate, tocopherols, ethoxyquinone, propyl gallate, rosemary extract, grapeseed extract, ascorbyl stearate, hydroquinone and mixtures thereof.
11. The encapsulated material of embodiment 6 which is a food, pharmaceutical, cosmeceutical or nutraceutical item.
12. The encapsulated material of embodiment 6, wherein said shell component is present in an amount of from about 20 to about 70 percent by weight of said core component.
13. The encapsulated material of embodiment 12, wherein the shell component has a thickness of from about 1 µm to about 1,000 µm.
14. The encapsulated material of embodiment 13, wherein the shell component has a thickness of from about 250 µm.
15. The encapsulated material of embodiment 6, wherein said antioxidant is present in an amount of about 0.005 to about 5 percent by weight of the coating component.
16. The encapsulated material of embodiment 15, wherein said antioxidant is present in an amount of about 0.02 to about 0.5 percent by weight of the coating component.
17. An encapsulated material, comprising: a core of omega-3-oil; and a shell component encapsulating said core component, wherein said shell component comprises a partially hydrogenated palm wax and L-ascorbic acid 6-palmitate.
18. A method of protecting a core material from damage, the method comprising encapsulating a core with the coating composition of embodiment 1.
19. The method of embodiment 18, wherein the coating thickness is from about 1 µm to about 1,000 µm.
20. The method of embodiment 19, wherein the coating thickness is from about 10 µm to about 250 µm.

The invention will be described further in the following examples, which are included for purposes of illustration and are not intended, in any way, to be limiting of the scope of the invention.

### EXAMPLES

### Example 1

### Formulation of Encapsulation 4

500g of a free flowing powder containing 50% omega-3 oil and 0.5% of silica flow aid (Syloid, GraceDavidson, Columbia, MD) was placed in a fluid bed coating chamber (FLM-1 Wurster Fluid Bed Accelerator, Vector Corporation, Marion IA) as the core material.

199.8g of partially hydrogenated palm wax (Stable Flake P, Cargill Foods) and 0.2g of L-ascorbic acid 6-palmitate (Alfa Aesar) were combined and melted at 110°C in a separate second container to form a coating mixture.

The coating mixture, kept at approximately 90C°, was attached to the feed line of the fluid bed chamber. The feed line was wrapped with heat tape to ensure the coating mixture was flowable. The coating mixture was sprayed at 10g/min into the chamber to encapsulate the core material. When 30% coating (based on the weight of the core material) was sprayed onto the core, the feed line was closed and the resultant encapsulants were removed from the chamber.

Encapsulants 1, 2 and 3 were made in the same manner. The coating components and the coating percent sprayed onto the core materials are listed in Table 1.

**Table 1**

| Encapsulant | | 1 | 2 | 3 | 4 |
|---|---|---|---|---|---|
| Core | Syloid¹ | 100% | 100% | 100% | 100% |
| Coating | Lipid-based polymer² | 100% | 99.95% | 99.95% | 99.90% |
| | Non-lipid-based anti-oxidant⁴ | -- | 0.05% | -- | -- |
| | Lipid-based anti-oxidane³ | -- | -- | 0.05% | 0.10% |
| Coating percent⁵ | | 50% | 30% | 30% | 30% |

| | | | | | |
|---|---|---|---|---|---|
| ¹ 50% Omega-3-oil and 0.5% silica flow aid, GraceDavidson, Columbia, MD ² Partially hydrogenated palm wax, Stable Flake P, Cargille Foods, NJ ³ L-Ascorbic acid 6-palmitate, Alfa Aesar, MA ⁴ Gravinol-T, Kikkoman Corporation, CA ⁵ {(coating wt used to coat the core) / (core wt)} x 100% | | | | | |

### Example 2

### Stability test

The stability of the encapsulant was determined by measuring the amount of free C-3 aldehydes from omega-3-fish oil, specifically acrolein and propanal using GC-FID (Hewlett Packard 5890 GC with Perkin Elmer HS40 headspace sampler, GC column - 30m.x0.53mm ID Stabilwax DA-1.5um film). Acrolein and propanal are generated by the oxidative breakdown of omega-3 fatty acids and their acyl esters.

100 mg of encapsulated sample and 1 mL of water containing 8.02ppm of C5 Alcohol (internal standard) were added into a headspace vial (National Scientific). The vial and its contents were heated at 80°C for 20 minutes prior to the injection into the GC-FID. The sample was compared to five external reference standards, ranging from 0.01 to 100 µg/g (ppm) of acrolein and propanal.

The encapsulated samples were placed in a temperature/humidity chamber set at 40°C and 43% relative humidity for an accelerated stability test. The encapsulated samples were tested weekly for the quantity of C-3 aldehydes. As the coating no longer sufficiently protected the core, omega-3 fish oil oxidized and produced C-3's. The oxidative damage of omega-3 fish oil (core) was determined by the rapid increase in the C-3 levels. Conversely, steady low levels of C-3 indicated that the coating sufficiently protected the omega-3 fish oil from oxidation. The stability results are shown in Figure I.

As shown in Figure I, the coating made from lipid-based polymer and lipid-based ant-oxidant (Encapsulants 3 and 4) protected the core from oxidation better than other coatings (Encapsulants 1 and 2) during the accelerated stability test. On the other hand, the C-3 levels of Encapsulant 1 (lipid-based polymer) increased rapidly starting week 3, and Encapsulant 2 (lipid-based polymer with non-lipid-based anti-oxidant) increased rapidly between week 5 and 6. The C-3 levels of Encapsulant 3 and 4, combination of lipid-based polymer with lipid-based anti-oxidant, remained low even at week 7. For Encapsulant 4, the C-3 levels remained low even at week 9 in condition of 40°C and 43% relative humidity. Hence, Encapsulant 3 and 4 protected the encapsulated core material from oxidation better than Encapsulants 1 and 2..

Many modifications and variations of this invention can be made without departing from its spirit and scope, as will be apparent to those skilled in the art. The specific embodiments described herein are offered by way of example only, and the invention is to be limited only by the terms of the appended claims, along with the full scope of equivalents to which such claims are entitled.

In the claims, the terms "consists" or "consisting of" is intended to mean excluding more than traces of other than the recited elements; the terms "consists essentially of" or "consisting essentially of" is intended to mean excluding other elements of any essential significance to the claimed combination; and the terms "comprises" or "comprising" is intended to mean including the following elements, but not excluding others.

## Claims

1. A coating composition comprising: a lipid polymer; and a lipid-based anti-oxidant, wherein the anti-oxidant is soluble in the lipid polymer.

2. The coating composition of claim 1 wherein the polymer is selected from the group consisting of waxes, partially hydrogenated oil, fats, gums, celluloses derivatives, proteins, synthetic polymer materials, alginate, carrageenan and mixtures thereof.

3. The coating composition of claim 1 or 2 wherein the anti-oxidant is selected from the group consisting of butylated hydroxyanisole (BHA), butylated hydroxytoluene (BHT), tert-butylhydroquinone (TBHQ), ascorbyl palmitate, tocopherols, ethoxyquinone, propyl gallate, rosemary extract, grapeseed extract, ascorbyl stearate, hydroquinone and mixtures thereof.

4. The coating composition of claim 3, wherein said antioxidant is present in an amount of about 0.005 to about 5 percent by weight of the lipid polymer.

5. The coating composition of claim 4, wherein said antioxidant is present in an amount of about 0.02 to about 0.5 percent by weight of the lipid polymer.

6. An encapsulated material, comprising: a core component; and a shell component encapsulating said core component, wherein said shell component comprises a lipid polymer material and a lipid-based anti-oxidant.

7. The encapsulated material of claim 6, wherein said core component is at least one of oxygen sensitive or water sensitive.

8. The encapsulated material of claim 7, wherein said core component is selected from the group consisting of unsaturated fatty acids, betacarotene, lutein, zeazanthin, iron salts, copper salts, selenium salts, flavonoids, coenzyme Q10, herbs, spices, flavorants, extracts, protein and peptide drugs, amino acids and amino acid residues, surfactants, enzymes, peroxides, fragrances, catalysts, vitamins, nutritional supplements, minerals, herbal products, food additives and mixtures thereof.

9. The encapsulated material of any one of claims 6-8 wherein said lipid polymer material is selected from the group consisting of waxes, partially hydrogenated oil, fats, gums, celluloses derivatives, proteins, synthetic polymer materials, alginate, carrageenan and mixtures thereof.

10. The encapsulated material of any one of claims 6-9, wherein said anti-oxidant is selected from the group consisting of butylated hydroxyanisole (BHA), butylated hydroxytoluene (BHT), tert-butylhydroquinone (TBHQ), ascorbyl palmitate, tocopherols, ethoxyquinone, propyl gallate, rosemary extract, grapeseed extract, ascorbyl stearate, hydroquinone and mixtures thereof.

11. The encapsulated material of any one of claims 6-10 which is a food, pharmaceutical, cosmeceutical or nutraceutical item.

12. The encapsulated material of any one of claims 6-11, wherein said shell component is present in an amount of from about 20 to about 70 percent by weight of said core component.

13. The encapsulated material of claim 12, wherein the shell component has a thickness of from about 1 µm to about 1,000 µm.

14. The encapsulated material of claim 13, wherein the shell component has a thickness of from about 250 µm.

15. The encapsulated material of any one of claims 6-14, wherein said antioxidant is present in an amount of about 0.005 to about 5 percent by weight of the coating component.

16. The encapsulated material of claim 15, wherein said antioxidant is present in an amount of about 0.02 to about 0.5 percent by weight of the coating component.

17. An encapsulated material, comprising: a core of omega-3-oil; and a shell component encapsulating said core component, wherein said shell component comprises a partially hydrogenated palm wax and L-ascorbic acid 6-palmitate.

18. A method of protecting a core material from damage, the method comprising encapsulating a core with the coating composition of any one of claims 1-5.

19. The method of claim 18, wherein the coating thickness is from about 1 µm to about 1,000 µm.

20. The method of claim 19, wherein the coating thickness is from about 10 µm to about 250 µm.
